## Europäisches Patentamt
## European Patent Office
## Office européen des brevets

(11) Veröffentlichungsnummer: **0 014 821**
**B1**

(12)
# EUROPÄISCHE PATENTSCHRIFT

(45) Veröffentlichungstag der Patentschrift:
**28.09.83**

(51) Int. Cl.³: **A 61 K 49/04**

(21) Anmeldenummer: **80100037.3**

(22) Anmeldetag: **04.01.80**

(54) **Flüssiges Präparat zur Verwendung bei der peroralen Schnellcholecystographie und Verfahren zu ihrer Herstellung.**

(30) Priorität: **19.02.79 DE 2906246**

(43) Veröffentlichungstag der Anmeldung:
**03.09.80 Patentblatt 80/18**

(45) Bekanntmachung des Hinweises auf die Patenterteilung:
**28.09.83 Patentblatt 83/39**

(84) Benannte Vertragsstaaten:
**BE CH DE FR GB IT LU NL SE**

(56) Entgegenhaltungen:
**DE-A-2 235 935**
**DE-A-2 505 218**

(73) Patentinhaber: **CHEMIE LINZ AKTIENGESELLSCHAFT, St. Peter-Strasse 25, A-4020 Linz (AT)**
(84) Benannte Vertragsstaaten: **BE CH FR GB IT LU NL SE**

(73) Patentinhaber: **Lentia Gesellschaft mit beschränkter Haftung, Arabellastrasse 4 Postfach 81 05 08, D-8000 München 81 (DE)**
(84) Benannte Vertragsstaaten: **DE**

(72) Erfinder: **Obendorf, Werner, Dr., Preglstrasse 18, A-4020 Linz (AT)**
Erfinder: **Schwarzinger, Ernst, Neuhoferstrasse 41, A-4020 Linz (AT)**
Erfinder: **Lindner, Irmgard, Dr., Novaragasse 8, A-4020 Linz (AT)**
Erfinder: **Takacs, Friedrich, Dr., Carl Boschweg 10, A-4020 Linz (AT)**

**Die Akte enthält technische Angaben, die nach dem Eingang der Anmeldung eingereicht wurden und die nicht in dieser Patentschrift enthalten sind.**

## Flüssiges Präparat zur Verwendung bei der peroralen Schnellcholecystographie und Verfahren zu ihrer Herstellung

Die Erfindung betrifft eine das nachstehend definierte flüssige Präparat zur Verwendung bei der peroralen Schnellcholecystographie, mit der man in einer beträchtlichen Anzahl der Fälle auch eine einwandfreie Darstellung der Gallengänge erhalten kann.

Für die Darstellung der Gallenblase im Röntgenbild stehen prinzipiell zwei Wege zur Verfügung, die Darstellung nach peroraler Verabreichung, bei der das Kontrastmittel über die Resorption aus dem Magen-Darmtrakt in die Gallenflüssigkeit kommt, und die nach intravenöser Verabreichung, also einer Applikation direkt in die Blutbahn. Die Perorale Cholecystographie, die an sich für den Patienten die geringere Belastung darstellt, wird in der Regel so durchgeführt, daß das Mittel am Vorabend der Untersuchung eingenommen wird. Sie hat den Nachteil, daß damit zwar die Gallenblase, nicht aber das Gallengangsystem sichtbar wird. Letzteres darzustellen war bisher der intravenösen Cholecystographie vorbehalten, bei der man aber die intravenöse Applikation einer stark jodhaltigen Substanz in die Vene in Kauf nehmen muß.

Es wurden auch schon Mittel gefunden, die nach peroraler Verabreichung wesentlich rascher resorbiert und über die Galle ausgeschieden wurden (DE-PS 1 467 996, AT-PS 275 025, DE-PS 2 235 915, DE-AS 2 235 935), so daß Verabreichung und Untersuchung an einem Tag, und zwar im Abstand von wenigen Stunden gemacht werden können.

Gemäß DE-AS 2 235 935 werden hierzu bestimmte trijodierte Aminobenzolcarbonsäuren, die in 3-Stellung durch eine Amidinogruppe substituiert sind, vorgeschlagen, und zwar unter anderem die Verbindung N-[3-(1'-3''-Oxapentamethylenamino-äthylidenamino)-2,4,6-trijodbenzoyl]-$\beta$-amino-$\alpha$-methylpropionsäure (Iomorinsäure). Als Darreichungsform für die perorale Schnellcholecystographie werden hierfür Tabletten, Dragees, Pulver oder Granulat vorgesehen, die die Säure oder Salze enthalten. Überhaupt wurde bisher für die orale Schnellcholecystographie Darreichungsformen der Vorzug gegeben, die ungelöst den Magen passieren. Die Darreichungsform der wäßrigen Lösungen von Salzen blieb hingegen für die intravenöse Applikation vorbehalten.

Bei der peroralen Schnellcholecystographie mit diesen bekannten Mitteln wurde bei pharmakologischen Untersuchungen an Katzen bereits gesehen, daß hier im Gegensatz zu bisher üblichen oralen Cholecystographie Gallengänge sichtbar wurden. Bei der praktischen Erprobung am Menschen, z. B. mit Tabletten auf Basis des oben erwähnten Na-Iomorinats, zeigt sich aber, daß jene Fälle, in denen eine ausreichende Diagnose des Gallengangsystem möglich war, bei unter 10%, meist rund um 5% der insgesamt untersuchten Fälle blieben.

Es konnte nun gefunden werden, daß der Prozentsatz der Fälle mit ausreichender Diagnosemöglichkeit der Gallengänge mittels oraler Schnellcholecystographie entscheidend, nämlich auf bis zu 50% der untersuchten Fälle angehoben werden kann, wenn man das Na-Iomorinat in seiner auf physiologischen pH gebrachten anspruchsgemäßen wäßrigen Lösung peroral verabreicht. Das war überraschend, da nicht zu erwarten war, daß die in wäßriger Lösung verabreichte Substanz rasch genug den Magen passiert, während bisher eine rasche unverteilte Magenpassage der gesamten Dosis als Voraussetzung für eine gute Darstellung der Gallengänge galt.

Die erfindungsgemäße Lösung unterscheidet sich insbesondere von den in der DE-AS 2 235 935 allgemein genannten Lösungen durch den speziellen pH-Wert und der Applikations-Dosiseinheit. Diese Auswahl wird durch den Stand der Technik in keiner Weise nahegelegt.

Gegenstand der vorliegenden Erfindung ist demnach eine wäßrige Lösung zur Verwendung bei der peroralen Schnellcholecystographie, die als Wirkstoff das Na-Salz der N-[3-(1'-3''-Oxapentamethylen-amino-äthylidenamino)-2,4,6-trijodbenzoyl]-$\beta$-amino-$\alpha$-methylpropionsäure (Iomorinat) enthält, und zwar in der Form eines flüssigen Präparats für die einmalige perorale Applikation zur Durchführung der Schnellcholecystographie, das dadurch gekennzeichnet ist, daß es aus einer abgeschlossenen Einheit von 20 bis 30 ml einer träger- und stabilisatorfreien wässerigen Lösung von mindestens 4 g und höchstens 6 g Na-Salz der Iomorinsäure besteht und einen pH-Wert von 7,4 bis 8 aufweist.

Die Herstellung der erfindungsgemäßen Lösung gelingt auf einfache Weise, indem Iomorinsäure in Gegenwart der stöchiometrischen Menge an NaOH in Wasser gelöst wird. Die anschließende Einstellung des pH-Wertes auf den physiologischen Bereich von 7,4 bis 8 geschieht dann durch Zugabe einer kleinen Menge verdünnter NaOH oder von einer geringen Menge physiologisch unbedenklicher Säuren, wie Salzsäure oder Essigsäure, oder von weiterer Iomorinsäure, worauf durch Zugabe von Wasser auf das gewünschte Volumen verdünnt wird.

Die Zugabe von Puffersubstanzen ist nicht erforderlich, da auf Grund des amphoteren Charakters der Iomorinsäure die Lösung ohne jeglichen Zusatz haltbar ist.

Die Dosis, mit der eine gute Diagnose erzielt wird, liegt bei 4 bis 6 g pro Patient. Die Konzentration der wäßrigen Lösung ergibt sich aus dem beanspruchten Bereich und ist für das Gelingen des Diagnose-Verfahrens nicht kritisch. Man kann daher die Konzentration innerhalb dieses Bereiches dem Volumen anpassen, das für eine Einzelverabreichung als zweckmäßig angesehen wird. So können z. B. 4,5 bis 5,5 g Na-Iomorinat zu einer Lösung eines Volumens von 20 bis 30 ml verarbeitet werden, eine Menge, die ein Patient mit einem Schluck zu sich nehmen kann.

Die erfindungsgemäße flüssige Form ist besonders gut verträglich und auch bei Patienten mit Schluckbeschwerden leicht applizierbar, was einen Vorteil bedeutet. Die Aufnahmen des Gallengangsystems sind dann etwa 60 bis 90 Minuten nach Applikation möglich, während zur Beurteilung der Gallenblase selbst meist noch eine zweite Aufnahme, etwa 3 bis 4 Stunden nach der Applikation, zu empfehlen ist. Damit kann doch einem beträchtlichen Prozentsatz jener Patienten, bei denen neben einer Untersuchung der Gallenblase auch eine solche des Gallengangsystems erforderlich ist, die Prozedur der intravenösen Verabreichung mit ihren möglichen allergischen Reaktionen erspart bleiben.

Das erfindungsgemäße flüssige Präparat bietet aber auch einen besonders vorteilhaften Darreichungsmodus. Verabreicht man nämlich das erfindungsgemäße Präparat in der für Diagnosezwecke erforderlichen Dosis nicht nur 60 bis 120 Minuten vor der ersten Röntgenaufnahme, sondern zusätzlich die für diagnostische Zwecke ausreichende Dosis des gleichen oder eines anderen oralen Gallekontrastmittels am Vorabend der Untersuchung, so ist es möglich, mit einer einzigen Röntgenaufnahme Gallenblase und Gallengänge gleichzeitig sichtbar zu machen. Dieser Darreichungsmodus bringt nicht nur eine wesentliche Arbeitsersparnis für das Klinikpersonal mit sich, sondern reduziert auch die Röntgenstrahlenbelastung des Patienten auf die Hälfte.

## Beispiel 1

0,245 g Natriumhydroxyd werden in etwa 14 ml Wasser gelöst und leicht erwärmt. Hierauf werden unter Rühren 4,365 g N-[3-(1'-3''-Oxapentamethylenamino-äthylidenamino)-2,4,6-trijodbenzoyl]-$\beta$-amino-$\alpha$-methylpropionsäure eingetragen und so lange gerührt, bis eine klare Lösung erhalten wird. Nach Feineinstellung des pH-Wertes auf 7,4 wird auf ein Volumen von 20 ml aufgefüllt. Die Lösung enthält 4,5 g Natriumsalz der oben angeführten Säure und entspricht einer Normaldosis für eine Untersuchung.

Bei der klinischen Prüfung wurde nach Verabreichung dieser Lösung an Patienten in nüchternem Zustand und Aufnahmen 60 Minuten und 4 Stunden nach Applikation im Durchschnitt von 700 Untersuchungen in 67% der Fälle ein Diagnostisch ausreichendes Bild der Gallenblase und in 39,1% der Fälle ein diagnostisch ausreichendes Bild der Gallengänge erhalten, das heißt also, daß in 58% der Fälle, in denen die Gallenblase sichtbar war, auch eine Diagnose der Gallengänge vorgenommen werden konnte.

## Patentanspruch

Flüssiges Präparat für die einmalige perorale Applikation zur Durchführung der Schnellcholecystographie, enthaltend ein Salz der N-[3-(1',3''-Oxapentamethylenaminoäthylidenamino)-2,4,6-trijodbenzoyl]-$\beta$-amino-alpha-methylpropionsäure (Iomorinsäure), dadurch gekennzeichnet, daß das Präparat aus einer abgeschlossenen Einheit von 20 bis 30 ml einer träger- und stabilisatorfreien wässerigen Lösungs von mindestens 4 g und höchstens 6 g Na-Salz der Iomorinsäure besteht und einen pH-Wert von 7,4 bis 8 aufweist.

## Claim

A liquid preparation for single oral administration and suitable for use in rapid cholecystography combined with cholangiography containing a salt of N-[3-(1',3''-oxapentamethyleneamino-ethylideneamino)-2,4,6-triiodobenzoyl]-$\beta$-amino-$\alpha$-methyl-propionic acid (iomorinic acid) characterized in that the preparation consists of a single dosage unit of 20 to 30 ml of an aqueous solution containing 4 to 6 g of the sodium salt of iomorinic acid, said solution being free of carriers and stabilizing agents and having a pH value in the range of 7.4 to 8.

## Revendication

Préparation liquide destinée à l'administration en une seule prise par voie orale, en vue d'effectuer une cholécystographie rapide, contenant un sel de l'acide N-[3-(1'-3''-oxapentaméthylèneamino-éthylidèneamino)2,4,6-triiodobenzoyl]-$\beta$-amino-$\alpha$-méthylpropionique (acide iomorinique), caractérisée en ce que la préparation est constituée par une unité complète constituée par 20 à 30 ml d'une solution aqueuse ne contenant ni excipient ni stabilisant d'au moins 4 g et d'au plus 6 g du sel de Na de l'acide iomorinique, et présente un pH de 7,4 à 8.